# EUROPEAN PATENT SPECIFICATION

(11) **EP 3 360 564 B1**
(45) Date of publication and mention of the grant of the patent: **08.07.2020**
(21) Application number: 18155427.0
(22) Date of filing: 06.02.2018
(51) Int. Cl.: A61P 35/00, A61K 38/00, A61K 39/00, C07K 16/00, C07K 14/47

(54) **NOVEL PROTEIN TRANSFECTION COMPOSITIONS AND USES THEREOF**
NEUARTIGE PROTEINTRANSFEKTIONSZUSAMMENSETZUNGEN UND VERWENDUNGEN DAVON
NOUVELLES COMPOSITIONS DE TRANSFECTION DE PROTÉINES ET LEURS UTILISATIONS

(30) Priority: 09.02.2017 US 201762456992 P
(43) Date of publication of application: 15.08.2018
(73) Proprietor: Chang Gung Memorial Hospital, Linkou, Taoyuan City 33305 (TW)
(72) Inventor: CHAO, Angel, 33305 Taoyuan City (TW); WANG, Tzu-Hao, 33305 Taoyuan City (TW); CHEN, Shuan-Hua, 33305 Taoyuan City (TW)
(74) Representative: Cabinet Becker et Associés

(56) References cited:
- KR-A- 20030 078 115
- US-A1- 2016 008 426
- DIEGO S. D'ASTOLFO ET AL: "Efficient Intracellular Delivery of Native Proteins", CELL, vol. 161, no. 3, 1 April 2015 (2015-04-01) , pages 674-690, XP55338589, AMSTERDAM, NL ISSN: 0092-8674, DOI: 10.1016/j.cell.2015.03.028
- VIVIEN MARX: "Cell biology: delivering tough cargo into cells", NATURE METHODS, vol. 13, no. 1, 1 January 2016 (2016-01-01), pages 37-40, XP055466125, ISSN: 1548-7091, DOI: 10.1038/nmeth.3693
- QIAO JIN ET AL: "Zwitterionic drug nanocarriers: A biomimetic strategy for drug delivery", COLLOIDS AND SURFACES. B, BIOINTERFACES, vol. 124, 1 December 2014 (2014-12-01), pages 80-86, XP055466189, NL ISSN: 0927-7765, DOI: 10.1016/j.colsurfb.2014.07.013

### SEQUENCE LISTING

<110> Chang Gung Memorial Hospital, Linkou
<120> NOVEL PROTEIN TRANSFECTION COMPOSITIONS AND USES THEREOF
<130> B2673EP
<150> US 62/456,992
   <151> 2017-02-09
<160> 4
<170> PatentIn version 3.5
<210> 1
   <211> 18
   <212> PRT
   <213> Artificial Sequence
<220>
   <223> BiP
<400> 1
   Met Lys Leu Cys Ile Leu Leu Ala Val Val Ala Phe Val Gly Leu Ser
   1               5                   10                  15
   Leu Gly
<210> 2
   <211> 590
   <212> PRT
   <213> Artificial Sequence
<220>
   <223> SIP1
<400> 2
   Met Glu Ser Gly Ser Pro Met Gly Glu Val Glu Ile Ser Arg Thr Ile
   1               5                   10                  15
   Arg Thr Asn Gly Arg Gly Gln Arg Gly Tyr Asp Trp Gln Cys Lys Arg
               20                  25                  30
   Pro Ile Arg Val Ala Glu Val Arg Ser Ser Leu His Ser Trp Ser Leu
           35                  40                  45
   Arg Trp Val Asn Glu Leu Lys Glu Lys Gly Asn Lys Ala Leu Ser Val
       50                  55                  60
   Gly Asn Ile Asp Asp Ala Leu Gln Cys Tyr Ser Glu Ala Ile Lys Leu
   65                  70                  75                  80
   Asp Pro His Asn His Val Leu Tyr Ser Asn Arg Ser Ala Ala Tyr Ala
                   85                  90                  95
   Lys Lys Gly Asp Tyr Gln Lys Ala Tyr Glu Asp Gly Cys Lys Thr Val
               100                 105                 110
   Asp Leu Lys Pro Asp Trp Gly Lys Gly Tyr Ser Arg Lys Ala Ala Ala
           115                 120                 125
   Leu Glu Phe Leu Asn Arg Phe Glu Glu Ala Lys Arg Thr Tyr Glu Glu
       130                 135                 140
   Gly Leu Lys His Glu Ala Asn Asn Pro Gln Leu Lys Glu Gly Leu Gln
   145                 150                 155                 160
   Asn Met Glu Ala Arg Leu Ala Glu Arg Lys Phe Met Asn Pro Phe Asn
                   165                 170                 175
   Met Pro Asn Leu Tyr Gln Lys Leu Glu Ser Asp Pro Arg Thr Arg Thr
               180                 185                 190
   Leu Leu Ser Asp Pro Thr Tyr Arg Glu Leu Ile Glu Gln Leu Arg Asn
           195                 200                 205
   Lys Pro Ser Asp Leu Gly Thr Lys Leu Gln Asp Pro Arg Ile Met Thr
       210                 215                 220
   Thr Leu Ser Val Leu Leu Gly Val Asp Leu Gly Ser Met Asp Glu Glu
   225                 230                 235                 240
   Glu Glu Ile Ala Thr Pro Pro Pro Pro Pro Pro Pro Lys Lys Glu Thr
                   245                 250                 255
   Lys Pro Glu Pro Met Glu Glu Asp Leu Pro Glu Asn Lys Lys Gln Ala
               260                 265                 270
   Leu Lys Glu Lys Glu Leu Gly Asn Asp Ala Tyr Lys Lys Lys Asp Phe
           275                 280                 285
   Asp Thr Ala Leu Lys His Tyr Asp Lys Ala Lys Glu Leu Asp Pro Thr
       290                 295                 300
   Asn Met Thr Tyr Ile Thr Asn Gln Ala Ala Val Tyr Phe Glu Lys Gly
   305                 310                 315                 320
   Asp Tyr Asn Lys Cys Arg Glu Leu Cys Glu Lys Ala Ile Glu Val Gly
                   325                 330                 335
   Arg Glu Asn Arg Glu Asp Tyr Arg Gln Ile Ala Lys Ala Tyr Ala Arg
               340                 345                 350
   Ile Gly Asn Ser Tyr Phe Lys Glu Glu Lys Tyr Lys Asp Ala Ile His
           355                 360                 365
   Phe Tyr Asn Lys Ser Leu Ala Glu His Arg Thr Pro Asp Val Leu Lys
       370                 375                 380
   Lys Cys Gln Gln Ala Glu Lys Ile Leu Lys Glu Gln Glu Arg Leu Ala
   385                 390                 395                 400
   Tyr Ile Asn Pro Asp Leu Ala Leu Glu Glu Lys Asn Lys Gly Asn Glu
                   405                 410                 415
   Cys Phe Gln Lys Gly Asp Tyr Pro Gln Ala Met Lys His Tyr Thr Glu
               420                 425                 430
   Ala Ile Lys Arg Asn Pro Lys Asp Ala Lys Leu Tyr Ser Asn Arg Ala
           435                 440                 445
   Ala Cys Tyr Thr Lys Leu Leu Glu Phe Gln Leu Ala Leu Lys Asp Cys
       450                 455                 460
   Glu Glu Cys Ile Gln Leu Glu Pro Thr Phe Ile Lys Gly Tyr Thr Arg
   465                 470                 475                 480
   Lys Ala Ala Ala Leu Glu Ala Met Lys Asp Tyr Thr Lys Ala Met Asp
                   485                 490                 495
   Val Tyr Gln Lys Ala Leu Asp Leu Asp Ser Ser Cys Lys Glu Ala Ala
               500                 505                 510
   Asp Gly Tyr Gln Arg Cys Met Met Ala Gln Tyr Asn Arg His Asp Ser
           515                 520                 525
   Pro Glu Asp Val Lys Arg Arg Ala Met Ala Asp Pro Glu Val Gln Gln
       530                 535                 540
   Ile Met Ser Asp Pro Ala Met Arg Leu Ile Leu Glu Gln Met Gln Lys
   545                 550                 555                 560
   Asp Pro Gln Ala Leu Ser Glu His Leu Lys Asn Pro Val Ile Ala Gln
                   565                 570                 575
   Lys Ile Gln Lys Leu Met Asp Val Gly Leu Ile Ala Ile Arg
               580                 585                 590
<210> 3
   <211> 23
   <212> PRT
   <213> Artificial Sequence
<220>
   <223> V5-His
<400> 3
   Gly Lys Pro Ile Pro Asn Pro Leu Leu Gly Leu Asp Ser Thr Arg Thr
   1               5                   10                  15
   Gly His His His His His His
               20
<210> 4
   <211> 99
   <212> PRT
   <213> Mouse
<400> 4
   Thr Lys Ala Met Asp Val Tyr Gln Lys Ala Leu Asp Leu Asp Ser Ser
   1               5                   10                  15
   Cys Lys Glu Ala Ala Asp Gly Tyr Gln Arg Cys Met Met Ala Gln Tyr
               20                  25                  30
   Asn Arg His Asp Ser Pro Glu Asp Val Lys Arg Arg Ala Met Ala Asp
           35                  40                  45
   Pro Glu Val Gln Gln Ile Met Ser Asp Pro Ala Met Arg Leu Ile Leu
       50                  55                  60
   Glu Gln Met Gln Lys Asp Pro Gln Ala Leu Ser Glu His Leu Lys Asn
   65                  70                  75                  80
   Pro Val Ile Ala Gln Lys Ile Gln Lys Leu Met Asp Val Gly Leu Ile
                   85                  90                  95
   Ala Ile Arg

## Description

### FIELD OF THE INVENTION

The present invention relates generally to the fields of protein transfection. More particularly, it concerns novel protein transfection compositions and methods of transfecting cells or inhibiting cancer cell growth with such compositions.

### BACKGROUND OF THE INVENTION

Advances in science and technology lead to the development of numerous recombinant proteins and peptides for research or clinical therapy. Protein transfection plays an important role in studying biological functions of proteins in living cells, such as protein-protein interactions, protein trafficking, or protein targeting by antibodies. Several commercial protein transfection reagents are currently available, such as PULSin™, ProteoJuice™, Xfect™, and BioPorter®. However, expensive prices attributed to the low utility rate of these commercial products.

Protein therapy holds promises in the treatment of many diseases. Currently, clinically efficacious protein therapeutics mainly target cell-surface or extracellular proteins. Potential protein therapeutic focusing the cytoplasmic or nuclear targets may have been missed, and thus hindering the development of protein drugs.

Accordingly, there is still a need for a more affordable and effective protein transfection compositions that have one or more of the following properties such as low cytotoxicity, high transfection efficiency, and ease in small or large scale preparation. The present invention addresses this need and other needs.

### BRIEF SUMMARY OF THE INVENTION

Accordingly, the present invention provides methods of delivering a protein into a cell, comprising the steps of (a) combining the protein with a protein transfection composition to form a mixture, wherein the protein transfection composition consist of a 4-(2-hydroxyethyl)-1-piperazineethanesulfonic acid (HEPES) buffer; and (b) contacting the cell in a minimal essential medium with the mixture of step (a) to transfect the cell with the protein.

The use of a protein transfection composition for the manufacture of a medicament for delivering or transfecting a therapeutic protein into a cell, wherein the protein transfection composition consists of a HEPES buffer, is described.

The invention will become more apparent when read with the accompanying figures and detailed description which follow.

### BRIEF DESCRIPTION OF THE DAWINGS

Illustrative embodiments of the present invention are described in detail below with reference to the following Figures:

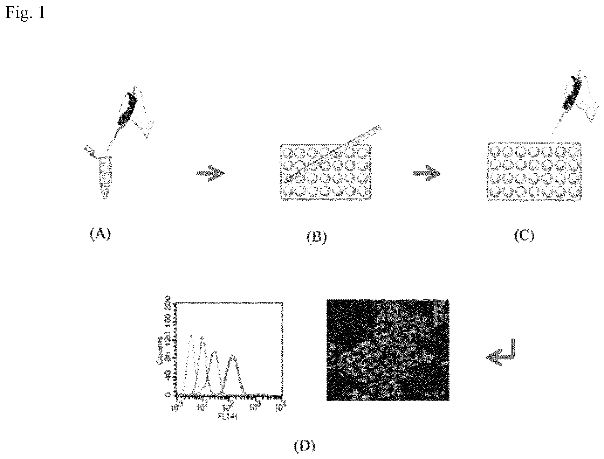

Fig. 1 schematically illustrates an embodiment of the protein transfection procedure.

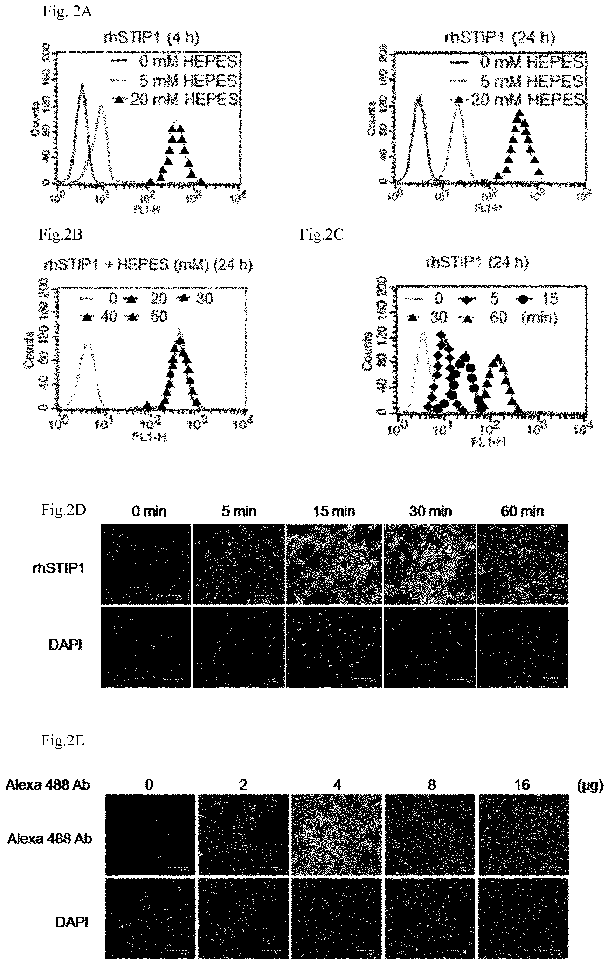

Fig. 2A to Fig. 2E are an assembly of images showing the effect of HEPES concentration, the incubation time, and volume ratio of protein to HEPES on protein transfection efficiency in ovarian cancer cells.

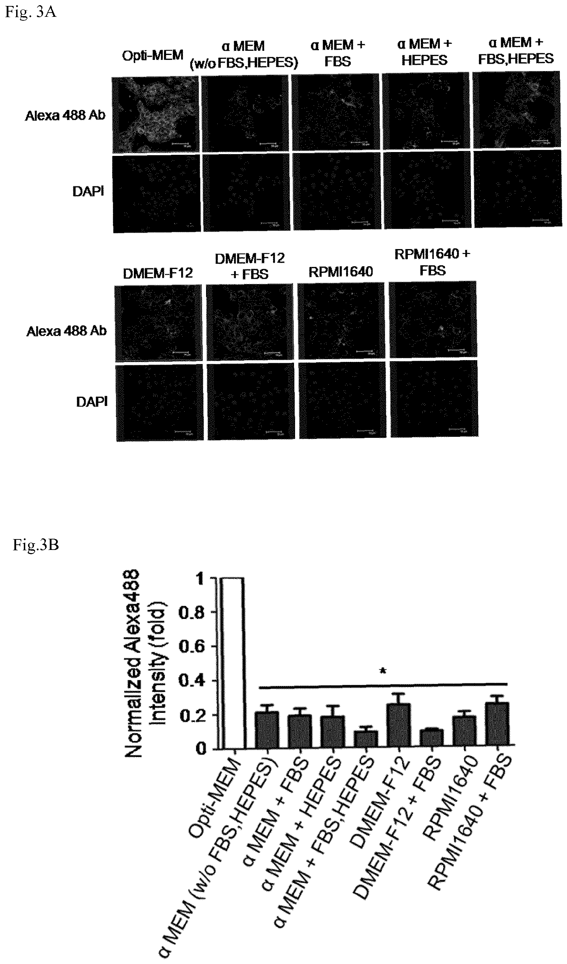

Fig 3A and Fig. 3B illustrate the effect of various cell culture mediums on protein transfection efficiency.

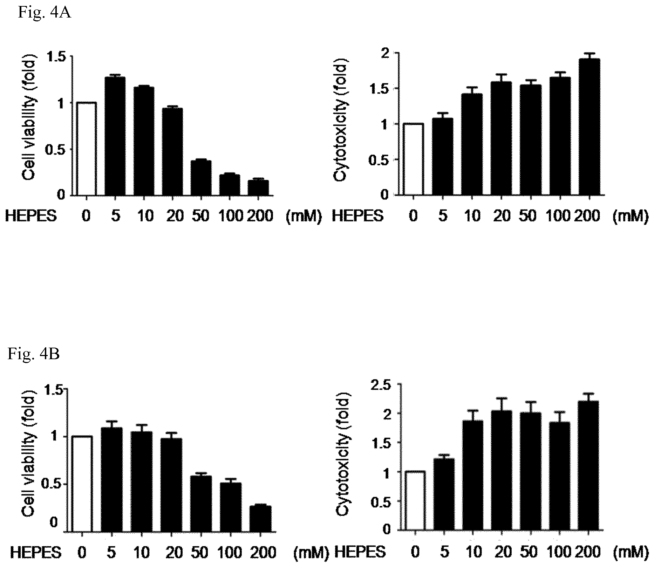

Fig. 4A and Fig. 4B are bar graphs illustrating the cytotoxicity of HEPES buffer on human ovarian cancer cells (Fig. 4A) and endometrial cancer cells (Fig. 4B).

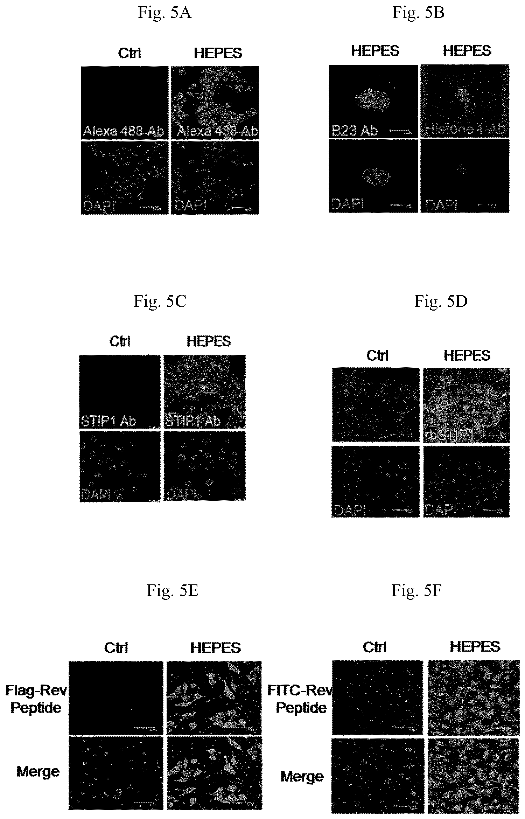

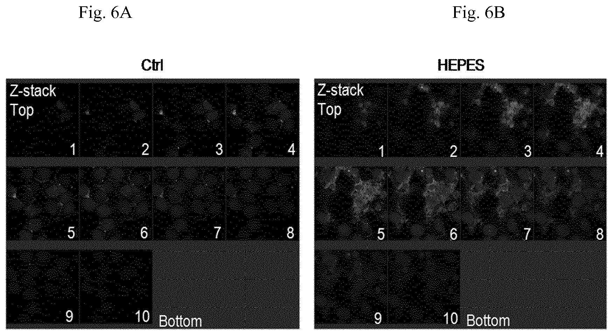

Fig. 5A to Fig. 5C and Fig. 6A and Fig. 6B are an assembly of immunofluorescent images showing the transfection of the following antibodies using an embodiment of the protein transfection composition used in the present invention: Alexa Fluor 488 anti-mouse IgG in ovarian cancer cells (MDAH2774) (Fig. 5A, Fig. 6A and 6B), anti-B23 antibody and anti-histone antibody in ovarian cancer cells (MDAH2774) (Fig. 5B) and anti-STIPl antibody in endometrial cancer cells (Fig. 5C). Fig. 5D to Fig. 5F are an assembly of immunofluorescent images showing the transfection of the following proteins using an embodiment of the protein transfection composition used in the present invention: v5-tagged recombinant human STIP1 proteins (rhSTIP1) in ovarian cancer cells (MDAH2774) (Fig. 5D), Flag-Rev peptide in ovarian (MOSEC) cancer cells (Fig. 5E) and FITC-Rev peptides in ovarian (MOSEC) cancer cells (Fig. 5F).

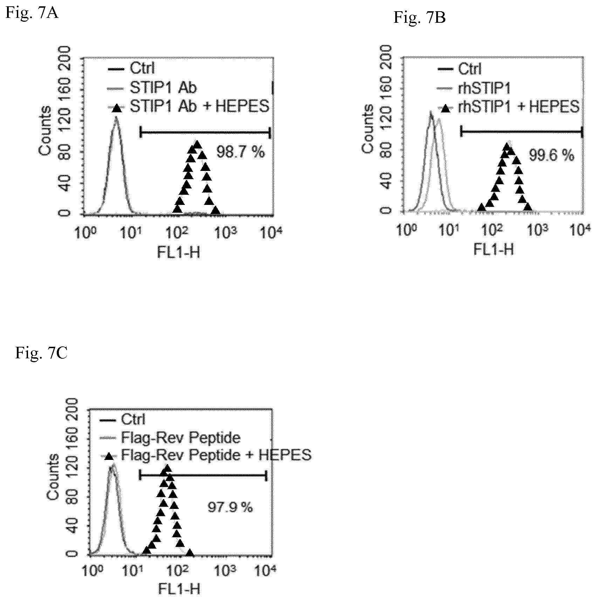

Fig. 7A to Fig. 7C are flow cytometry images showing the transfection efficiency of STIP1 antibody (Fig. 7A), rhSTIP1 protein (Fig. 7B), and Flag-Rev peptide (Fig. 7C) in ovarian (MDAH2774) cancer cells using an embodiment of the protein transfection composition used in the present invention.

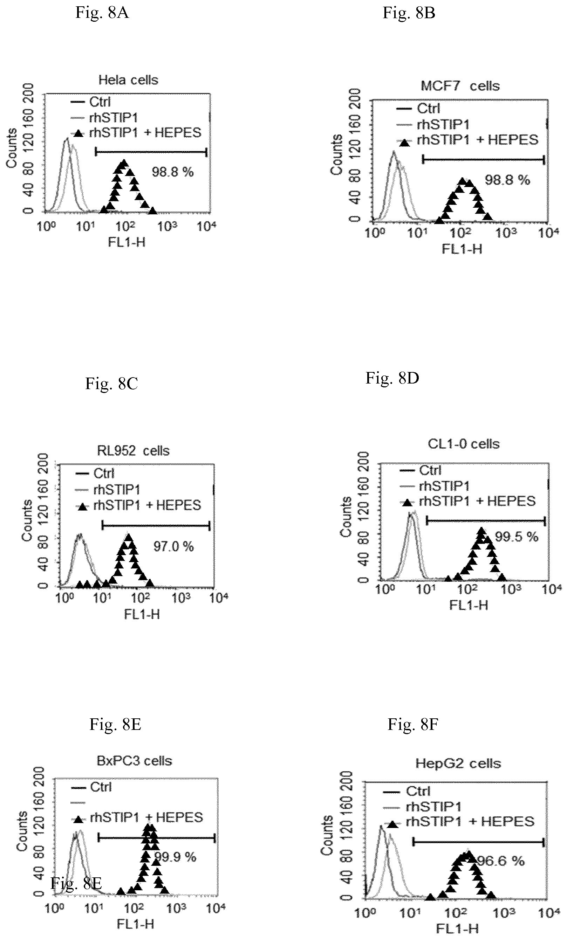

Fig. 8A to Fig. 8F are an assembly of flow cytometry images illustrating the transfection efficiency of shSTIP1 protein with or without an embodiment of the protein transfection composition used in the present invention in cervical cancer cells (Fig. 8A), breast cancer cells (Fig. 8B), endometrial cancer cells (Fig. 8C), lung adenocarcinoma cells (Fig. 8D), pancreatic cancer cells (Fig. 8E), and hepatocellular carcinoma cells (Fig. 8F).

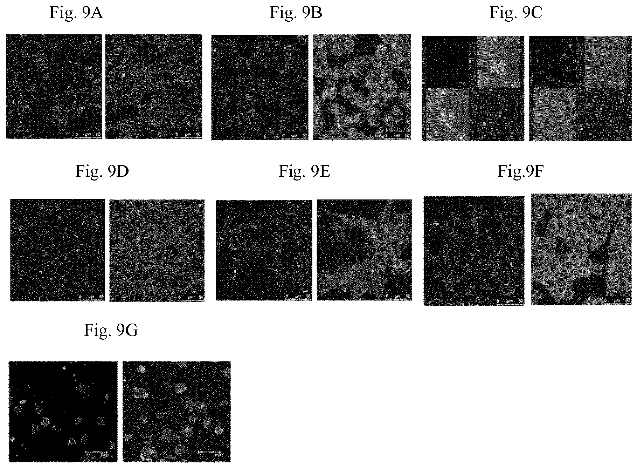

Fig. 9A to Fig. 9F are immunofluorescent staining showing the transfection of Alexa Fluor 488 anti-mouse IgG using an embodiment of the protein transfection composition used in the present invention in scarcoma cells (Fig. 9A), renal cell carcinoma (Fig. 9B), non Hodgkin's lymphoma (Fig. 9C), nasopharyngeal carcinoma (Fig. 9D), brain tumor (Fig. 9E), colon cancer (Fig. 9F), and leukemia cells (Fig. 9G).

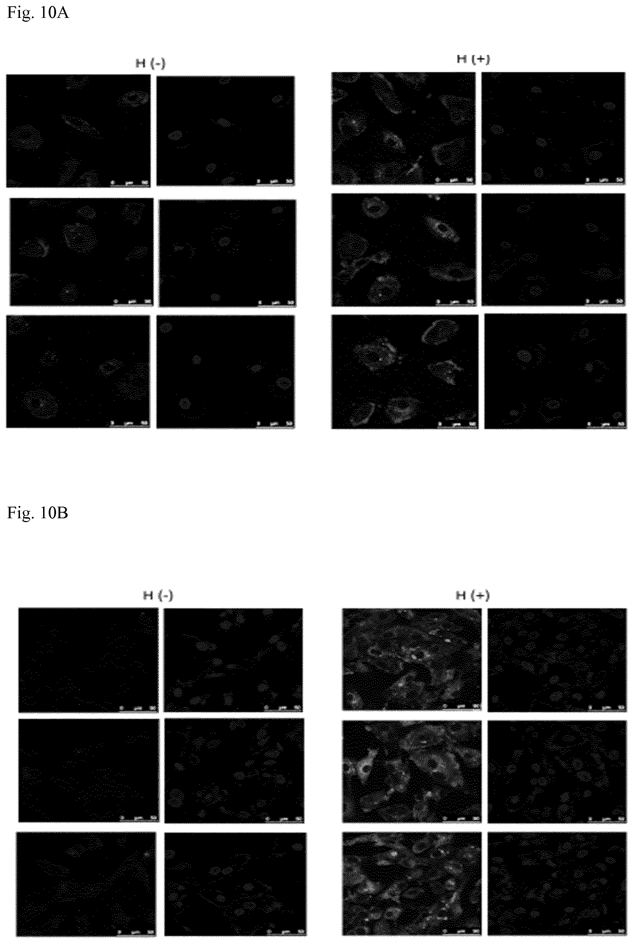

Fig. 10A and Fig. 10B are immunofluorescent staining which shows the transfection of Alexa Fluor 488 anti-mouse IgG with (H(+)) or without (H(-)) an embodiment of the protein transfection composition used in the present invention in breast cancer cells (BRCA009) after 6 hours of incubation (Fig. 10A) and in breast cancer cells (BRCA011) after 24 hours of incubation (Fig. 10B).

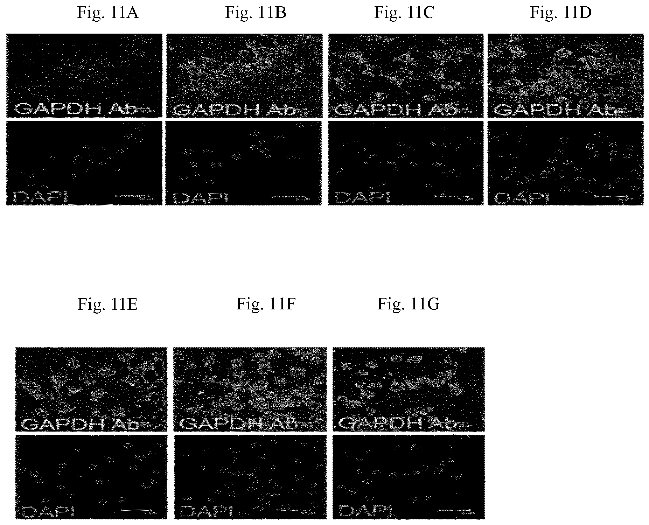

Fig. 11A to Fig.11G are an assembly of immunofluorescent images showing the transfection efficiency of anti-GAPDH antibody in human ovarian cancer cells using an embodiment of the protein transfection composition used in the present invention (Fig 11 G) and other protein transfection kits such as PULSin™ (Fig. 11B), ProteoJuice™ (Fig. 11C), Pro-Ject™ (Fig. 11D), Xfect™ (Fig. 11E), and BioPorter® (Fig. 11F). Fig. 11A is the control group.

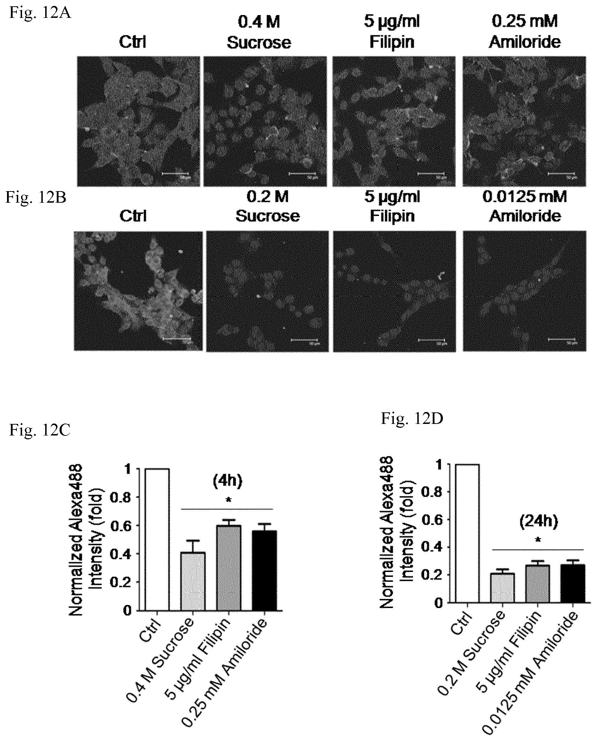

Fig. 12A to Fig. 12D illustrate antibody transfection using an embodiment of the protein transfection composition used in the present invention in ovarian cancer cells is reduced by sucrose, filipin, and amiloride after 4 hours (Fig. 12A and Fig. 12C) and 24 hours (Fig. 12B and Fig. 12D).

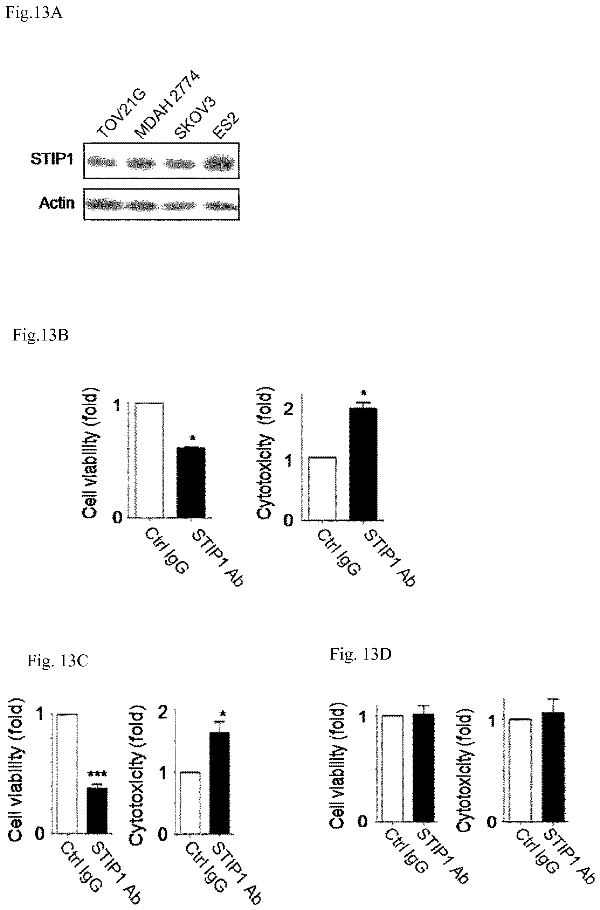

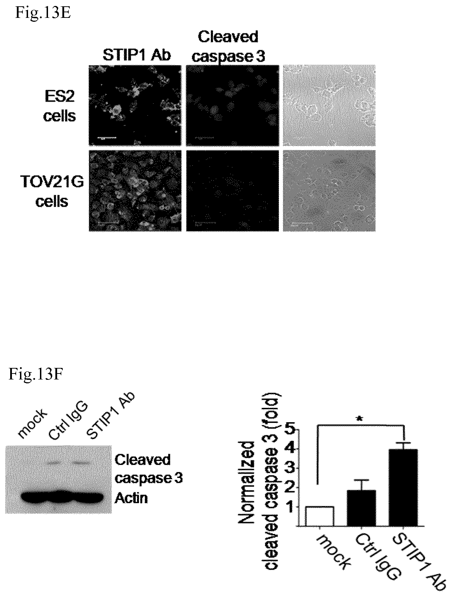

Fig. 13A illustrates different STIP1 expressions in various ovarian cancer cell lines (TOV21G, MDAH2774, SKOV3 and ES2). STIP1 antibody transfection using an embodiment of the protein transfection composition used in the present invention significantly inhibit cell growth and induced apoptosis in high STIP1 expression ovarian cancer cells compared to the control group (Fig. 13B for MADAH 2774 and Fig. 13C for ES2), but STIP1 antibody transfection induced less inhibition and apoptosis in lower STIP expression ovarian cancer cells (Fig. 13D for TOV21G). Fig. 13E and Fig. 13F show STIP1 antibody transfection using an embodiment of the protein transfection composition used in the present invention in ovarian cancer cells lead to a higher expression of caspase 3 in high STIP1 expression ovarian cancer cells (Fig. 13E for ES2 and Fig. 13F for MDAH2774) compared to that of low STIP1 expression ovarian cancer cells (Fig. 13E for TOV21G).

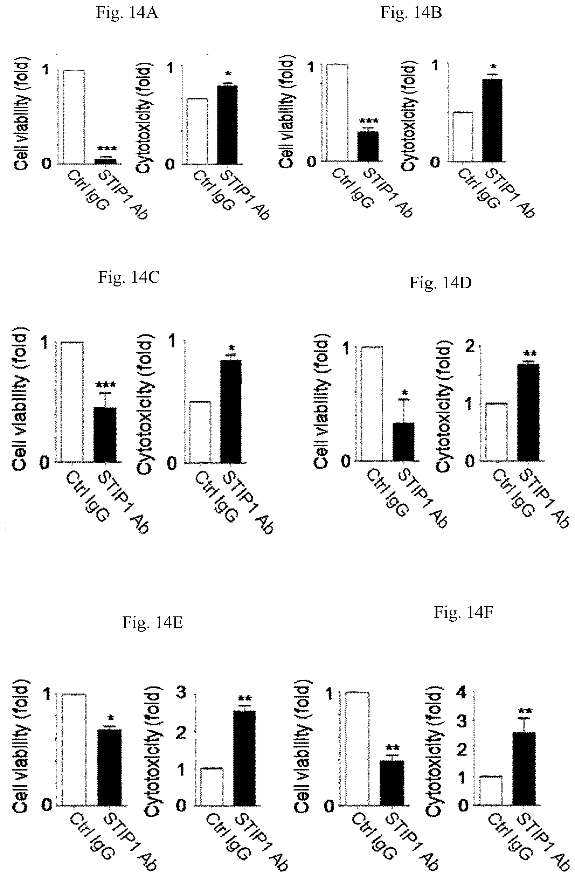

Fig. 14A to Fig. 14F are bar graphs showing the transfection of STIP1 antibody using an embodiment of the protein transfection composition used in the present invention suppressed cell viability and led to cytotoxicity in endometrial cancer (Fig. 14A), pancreatic cancer (Fig. 14B), head and neck cancer (Fig. 14C), lung cancer (Fig. 14D), breast cancer5 (Fig. 14E), and colon cancer (Fig. 14F).

### DETAILED DESCRIPTION OF THE INVENTION

### Definitions

As employed above and throughout the disclosure, the following terms, unless otherwise indicated, shall be understood to have the following meanings.

As used herein, the singular forms "a," "an," and "the" include the plural reference unless the context clearly indicates otherwise.

The term "treating," "treated," or "treatment" as used herein includes preventative (e.g. prophylactic), palliative, and curative uses or results.

The term "inhibiting" and "reducing" includes slowing, preventing or stopping the growth of.

The term "subject" can refer to a vertebrate having cancer or to a vertebrate deemed to be in need of cancer treatment. Subjects include warm-blooded animals, such as mammals, such as a primate, and, more preferably, a human. The term subject includes domesticated animals, such as cats, dogs, etc., livestock (for example, cattle, horses, pigs, sheep, goats, etc.) and laboratory animals (for example, mouse, rabbit, rat, gerbil, guinea pig, etc.). Thus, veterinary uses and medical formulations are contemplated herein.

All numbers herein may be understood as modified by "about." As used herein, the term "about," when referring to a measurable value such as an amount, a temporal duration, and the like, is meant to encompass variations of ± 10%, preferably ± 5%, more preferably ±1%, and even more preferably ± 0.1% from the specified value, as such variations are appropriate to % of cells transfected with the protein unless otherwise specified. As used herein, the term "about," when referring to a range, is meant to encompass variations of ± 10% within the difference of the range, preferably ± 5%, more preferably ± 1%, and even more preferably ± 0.1% from the specified value, as such variations are appropriate to the incubation time, unless other specified.

The term protein may include an intracellular protein such as proteins in the cytoplasm or nucleus, cell-surface protein, polypeptide, antibody, protein-nucleic acid conjugate, peptide-nucleic acid conjugate, fusion protein, recombinant protein, synthetic peptide, protein-nanoparticle conjugate, protein-polymer conjugate, conjugate between a protein-organic chemical entity or protein-inorganic chemical entity, multi-protein complexes, or any amino-acid containing moiety. In one embodiment, the protein-nanoparticle conjugate includes a protein encapsulated in a liposome. It is possible according to the invention to deliver proteins of different molecular masses, as discussed further below.

In one embodiment, the protein is about 1 kilodaltons to about 200 kilodaltons, or any value or range of values therebetween in 1 kilodalton increments (e.g., less than 5 kilodaltons, 50 kilodaltons, 150 kilodaltons, etc.)

Non-limiting examples of intracellular proteins include, but are not limited to: oxidoreductases, transferases, hydrolases, lyases, isomerases, ligases, kinases, phosphoproteines, and mutator transposons, DNA or RNA associated proteins (for example, homeobox, HMG, PAX, histones, DNA repair, p53, RecA, robosomal proteins, etc.), electron transport proteins (for example, flavodoxins); adaptor proteins; initiator caspases, effector caspases, inflammatory caspases, cyclins, cyclin-dependent kinases, cytokeletal proteins, G-protein regulators, small G proteins, mitochondria-associated proteins, PDZ adaptor proteins, PI-4-kinases, etc. Recombinant proteins of known function (such as rhSTIP1) or unknown functions are also included.

Antibodies include, but are not limited to, antigen-specific antibodies such as anti-STIPl antibodies, monoclonal and polyclonal antibodies, intact antibodies or single-chain antibodies.

Minimal essential medium ("MEM") is a synthetic culture medium comprising amino acids, salts, glucose and vitamins. Non-limiting examples include Opti-MEMS, α MEM, RPMI, DMEM, EMEM, and GMEM. A preferred example of MEM contains insulin, transferrin, hypoxanthin, and thymidine, such as the Opti-MEM®.

In an embodiment, the minimal essential medium further contains less than about 15%, 14%, 13%, 12%, 11%, 10%, 9%, 8%, 7%, 6%, 5%, 4%, 3%, 2%, 1%, or 0 % of fetal bovine serum ("FBS"), or any value or range of values therebetween in 0.1% increments (e.g., 0.1%. 1.8% , 0.2-2.8% etc.). In another embodiment, the minimal essential medium is substantially free of FBS.

The present invention provides method of introducing or delivering proteins into cells, comprises contacting the protein of interest with a protein transfection composition and incubating for about 15 to about 30 minutes to form a mixture, followed by contacting the mixture with the cells in a minimal essential medium. Without being bound by any particular theory, it is believed the protein transfection composition promotes the delivery or introduction of the protein into the cell by one or more of the following pathways: clathrin-mediated endocytosis, caveolae-mediated endocytosis, or macropinocytosis.

The method described herein may be used to deliver protein, therapeutic proteins, antibody (such as anti-STIPl antibody) *in vitro* or *in vivo.*

The invention may be used to deliver proteins to a variety of cells, including but not limited to cells in suspension, adherent cells, cancer cells, and primary cells. In one embodiment, the cell is STIP1 expressing cancer cell, selected from the group consisting of ovarian cancer cell, cervical cancer, endometrial cancer cell, lung cancer cell, pancreatic cancer cell, liver cancer cell, sarcoma cell, nasopharyngeal cancer cell, colon cancer cell, renal cancer cell, brain cancer cell, thyroid cancer cell, prostate cancer cell, skin cancer cell, leukemia cell and lymphoma cell.

It is further described the use of a protein transfection composition for the manufacture of a medicament for delivering or transfecting a therapeutic protein into a cell, wherein the protein transfection composition consists of a HEPES buffer, is provided. In this further aspect, a kit comprising a therapeutic protein and a protein transfection composition is also provided.

According to the invention, the protein transfection composition consists of a HEPES buffer.. In a particular embodiment, the protein transfection composition is substantially free of calcium phosphate.

FIG. 1 depicts the method of protein transfection in its simplest form: (A) proteins of interest and a protein transfection composition, such as HEPES buffer, are mixed and incubated for about 15 min to about 30 min ("the first incubation time") to form a mixture. (B) Cells to be transfected are cultured with a first cultured medium comprising at least about 10 % fetal bovine serum for abou 24 hours, followed by the replacement of the first cultured medium with a minimal essential medium. (C) The mixture in step (A) is added to the cells in minimal essential medium in Step (B) and incubated at 37°C for about 4 to about 24 hours ("the second incubation time"). (D) The protein transfection efficiency can be analyzed using various methods, such as immunofluorescent staining and flow cytometry.

Several parameters may be varied to increase protein transfection efficiency, such as the concentration of HEPES buffer, the first incubation time, the second incubation time, and the volume ratio of protein:HEPES buffer.

In one embodiment, the concentration of HEPES buffer is less than about 50 mM, 45 mM, 40 mM, 35 mM, 30 mM, 25 mM, 20 mM, 15 mM, 10 mM, 5 mM, 1 mM, or any value or range of values therebetween in 1 mM increments (e.g., about 5 mM, about 15 mM-35 mM, about 15-40 mM, about 20-30 mM, about 20-45 mM, about 10-50 mM, 20-50 mM, tc.).

In another embodiment, the first incubation time is about 15 to about 30 minutes. In another embodiment, the second incubation time is about 4 to about 24 hours. In yet another embodiment, the volume ratio of protein:HEPES buffer is about 1:5, about 1:10, about 1:20, about 1:30, about 1:40, about 1:50, about 1:60, about 1:70, about 1:30 to 1:70, about 1:35 to 1:65, about 1:40 to 1:60 or about 1:45 to 1:55.

The following examples further illustrate the present invention. These examples are intended merely to be illustrative of the present invention and are not to be construed as being limiting.

The following cell lines used in the examples were cultured in DMEM/F12 medium: Human ovarian cancer cell line, MDAH2774; mouse ovarian cancer cell line, MOSEC; human cervical cancer cell line, Hela; human breast cancer cell line, MCF7; human endometrial cancer cell line, RL952; human lung adenocarcinoma cell line, CL1-0. Human pancreatic cancer cell line, BxPC3 and human endometrial cancer cell line, ARK2, were cultured in RPMI 1640 medium. Human hepatocellular carcinoma cancer cell line, HepG2, was cultured in DMEM medium. All cells were cultured in medium with 10 % fetal bovine serum and appropriate amounts of penicillin and streptomycin. All media and Opti-MEM were purchased from Invitrogen (Life Technologies, Carlsbad, CA, USA).

Antibodies used in the study were Alexa Fluor 546 anti-mouse IgG, Alexa Fluor 488 anti-mouse IgG, and v5 antibody (commercially available from Life Technologies, Carlsbad, CA, USA); histone 1 antibody, and GAPDH antibody (commercially available from Santa Cruz Biotechnology, Dallas, TX, USA); flag antibody (commercially available from Sigma, St. Louis, MO, USA); STIP1 antibody (SEQ ID NO:4, commercially available from Abnova, Taipei, Taiwan); caspase 3 (commercially available from Cell Signaling Technology, Danvers, MA, USA); rabbit polyclonal anti-B23 antibodies(commercially available from Santa Cruz Biotechnology, Dallas, TX, USA).

The production and purification of rhSTIP1 (BiP-STIP1-V5-His, see SEQ ID NO:1, SEQ ID NO:2, and SEQ ID NO:3 respectively) was reported previously by Wang, T.H. *et al.* in Stress-induced phosphoprotein 1 as a secreted biomarker for human ovarian cancer promotes cancer cell proliferation. Mol Cell Proteomics 9, 1873-84 (2010). Briefly, rhSTIP1 was fused with two tags, 6x-His and v5, purified or detected by His or v5 antibodies. Flag-Rev peptide was composed of flag tag, Rev signal peptide, and 13 amino acids corresponding to B23 protein.

Chemicals were obtained from commercial sources as follows: sucrose and HEPES buffer: Sigma-Aldrich (Sigma, St. Louis, MO, USA), filipin (Santa Cruz Biotechnology, Dallas, TX, USA), and amiloride hydrochloride (Toronto Research Chemicals, Toronto, Ontario, Canada).

Immunofluorescent staining procedure: After protein transfection is completed, the cells in the culture dish were washed three times with phosphate buffered saline (PBS) and fixed with 4% paraformaldehyde for 20 min. The cells were washed again with PBS, perforated with 0.1% Triton X-100 for 5 min, and blocked with 10% BSA for 30 min. For Alexa Fluor 488 anti-mouse IgGs, cells were stained with DAPI for 10 min. For B23, histone 1 or GAPDH antibody transfection, cells were stained with Alexa Fluor 488 or 546 anti-mouse IgG antibodies for 30 min and then stained with DAPI. For rhSTIP1 proteins or Flag-Rev peptides transfection, cells were stained with anti-v5 or anti-flag antibodies, respectively, and then stained with Alexa Fluor 488 anti-mouse IgG and DAPI. All images were detected by confocal microscopes (Leica, Wetzlar, Germany). The fluorescent signals were analyzed and quantified by Q-Win software (Leica, Wetzlar, Germany).

Flow cytometry: After protein transfection was completed, the cells in the culture dish were washed with PBS, trypsinized and pelleted. The cells were mixed with Fixation/Permeabilization (eBioscience, Inc., San Diego, CA, USA) working solution and incubated at 4°C for 30 min to 24 h. Cells were washed twice with IX Permeabilization Buffer (eBioscience, Inc., San Diego, CA, USA). To assess the protein transfection efficiency, the cells were stained with anti-v5 or anti-flag antibodies and Alexa Fluor 488 anti-mouse antibodies on ice. Cells were suspended in IX PBS and analyzed by flow cytometry (BD Biosciences, Qume Drive San Jose, CA, USA).

Western blotting: Cell lysates were lyzed with RIPA buffer (150 mMNaCl, 20 mMTris-HCl (pH 7.5), 1% Triton X-100, 1% NP-40, 0.1% SDS, 0.5% deoxycholate) and freshly added with proteinase and phosphatase inhibitors (Bionovas, Toronto, Canada). The concentration of protein was determined by Bradford reagent (Bio-Rad, CA, USA). A total 100 µg proteins of each sample was electrophoresed in 10% SDS-PAGE and transferred to nitrocellulose membranes. All antibodies were from commercial sources and corresponding horseradish peroxidase-conjugated antibodies (Santa Cruz Biotechnology, Dallas, TX, USA). Labeled proteins were subsequently detected by enhanced chemiluminescence (Millipore, Bedford, MA, USA).

MTT and LDH assay: In MTT assay, the cells were incubated for 24 h and then centrifuged at 1500 rpm for 5 min. The supernatants were discarded and attached cells were analyzed by MTT reagent (Promega Corporation, Wisconsin, US). In LDH assay, the cells were incubated for 48 h and then centrifuged at 1500 rpm for 5 min. The supernatants were collected and analyzed by LDH reagent (Sigma, St. Louis, MO, USA). The cell viability (MTT assay) and cytotoxicity (LDH assay) were assayed following the manufacturer's protocol. The MTT and LDH signals were detected by ELISA Reader (Tecan Systems, Inc., San Jose, CA, USA).

### Example 1: Protein Transfection Procedure

Fig. 1 schematically illustrates the protein transfection procedure. Briefly, the protein of interest and a protein transfection composition, such as HEPES buffer, were mixed and incubated for about 15 min to about 30 min to form a mixture (Panel A). Cells to be transfected were replaced in a minimal essential medium (Panel B). The mixture in Panel A was added to the cells in Panel B and incubated at 37°C for about 4 to about 24 hours (Panel C). The protein transfection efficiency can be analyzed using immunofluorescent staining or flow cytometry (Panel D).

### Example 2: Incubation time and concentration of HEPES and the ratio of protein/HEPES are critical in transfection procedures

An *in vitro* evaluation of the optimal protein transfection condition was performed. According to the steps in Fig. 1. rhSTIP1 was incubated with HEPES buffers of different concentrations (5 or 20 mM) or Opti-MEM (control) at room temperature for about 15 minutes to form the protein mixtures to transfect human ovarian cancer cells. The mixtures were added to the cell culture dish comprising Opti-MEM and incubated at 37°C for about 4 to about 24 hours.

Fig. 2A shows a protein transfection efficiency increases with a higher HEPES concentration. Fig. 2B shows protein transfection efficiency does not increase further with HEPES concentration higher than 30 to 50 mM in ovarian cancer cells.

Fig. 2A further illustrates a longer second incubation time of 24 hours improved the protein transfection efficiency of 5 mM HEPES buffer compared to a shorter second incubation time of 4 hours.

Different amounts of Alexa Fluor® 488 anti-mouse IgG (0, 2, 4, 8, or 16 µg) were incubated with 20 mM HEPES buffer and mixed with ovarian (MDAH2774) cancer cells in Opti-MEM. Fig. 2E shows 4 µg IgG resulted in the highest transfection efficiency and the optimal volume ratio of protein:HEPES buffer is about 1:50.

### Example 3: Opti-MEM is the best transfection medium in protein transfection

Alexa Fluor 488 anti-mouse IgG antibodies were mixed with 20 mM HEPES to form a mixture. The mixture was incubated with human ovarian cancer cells in the following minimal essential media: Opti-MEM, α MEM (without HEPES or with additional 20 mM HEPES), DMEM-F12 (with 15 mM HEPES) or RPMI1640 (with 25 mM HEPES) in the presence and absence of 10% FBS for 24 hours. Protein transfection efficiency was assessed using confocal fluorescent microscope.

Fig. 3A are microscopic images showing the fluorescence signals were detected in cells incubated with all of the minimal essential media, with or without FBS, although Opti-MEM shows the highest transfection efficiency. Fig. 3B is a bar graph illustrating the fluorescent signals in Fig. 3A quantified by Q-Win software, expressed as a relative ratio (error bars indicate s.e.m. (n = 3) and asterisk denotes significant difference with P<0.001).

### Example 4: Cytotoxicity of Various HEPES Buffer Concentrations

The cytotoxicity of various HEPES buffer concentrations was evaluated. Human ovarian cancer cells and endometrial cancer cells were incubated in Opti-MEM and HEPES buffer (0-200 mM) for 24 h. Cell viability and cytotoxicity were analyzed by MTT and LDH assay, respectively.

Fig. 4A and Fig. 4B are bar graphs illustrating the cytotoxicity of HEPES buffer (0 mM to 200 mM) in human ovarian cancer cells (Fig. 4A) and endometrial cancer cells (Fig. 4B). Cell growth was inhibited when the concentration of HEPES buffer reached 50 mM. The final concentration of HEPES was 3.3 mM (200 µl 50 mM HEPES in 3 ml Opti-MEM for a 6-well plate), which is lower than 50 mM.

### Example 5: HEPES buffer can be used for antibody, recombinant protein, and small peptide transfection

The following proteins were incubated with Opti-MEM (control group) or 20-30 mM HEPES buffer to transfect ovarian cancer cells (MDAH2774 or MOSEC) or endometrial cancer cells: Alexa Fluor 488-conjugated, anti-mouse IgG antibody (about 150 kDa), anti-B23 antibody, anti-histone 1 antibody, anti-STIPl antibody, rhSTIP1 protein (62.6 kDa), Flag-Rev peptide (<5kDa) and FITC-Rev peptide (<5kDa). The transfection was carried out according to the steps in Example 1. Protein transfection efficiency was assessed by staining the transfected cancer cells with Alexa Fluor 488 or 594 anti-mouse IgG antibodies (for Alexa Fluor 488-conjugated anti-mouse IgG antibody, anti-B23 antibody, anti-histone 1 antibody, anti-STIPl antibody), v5 antibody (for rhSTIP1), or anti-Flag antibody (for Flag-Rev peptide), and fluorescent signals were analyzed with confocal microscopy.

HEPES buffer increased the transfection of Alexa Fluor 488 anti-mouse IgG antibody in the cytoplasm of the ovarian cancer cells when compared to the control group, illustrated by the increased fluorescent signals in Fig. 5A and Fig. 6A-6B (Z-stack mode from the top view to bottom position by confocal microscope

HEPES buffer can be used to transfect antibodies against nuclear proteins, such as anti-B23 antibody and anti-Histone 1 antibody. Fig. 5B illustrates ectopic fluorescent signals were detected in nucleus.

Antibodies against the cytoplasmic proteins, such as anti-STIPl antibody, was detected in the cytoplasm of ovarian cancer cells using HEPES buffer for transfection, see Fig. 5C. The transfection efficiency of anti-STIPl antibodies using HEPES buffer was measured using flow cytometry and anti-STIPl antibody signal was detected in 98.7% of the ovarian cancer cells (Fig. 7A).

HEPES buffers can be used to transfect proteins of various sizes, such as rhSTIP1 (62.6 kDa), Flag-Rev peptide (<5kDa), and FITC-Rev peptide (<5kDa), in ovarian cancer cells (MOSEC or MDAH2774). As illustrated in Fig. 5D-5F, the fluorescence signals of rhSTIP1, Flag-Rev peptide, and FITC-Rev peptide were detected in ovarian cancer cells using HEPES buffer. The transfection efficiency of rhSTIP1, Flag-Rev peptide using HEPEF buffer was measured using flow cytometry. Fig. 7B shows rhSTIP1 signal was detected in 99.6% of ovarian cancer cells and Fig. 7C shows Flag-Rev peptide signal was detected in 97.9% of the ovarian cancer cells.

Successful delivery of 5kDa peptide, 62 kDA rhSTIP1 protein and 150 kDa IgG antibody confirms that Zwitterionic buffers, such as HEPES buffer, can be used to transfect protein of different sizes.

### Example 6: HEPES buffer can be used to transfect protein in various cancer cells

Alexa Fluor 488 anti-mouse IgG or rhSTIP1 were incubated with Opti-MEM (the control group) or HEPES buffer (20 mM - 40mM) to transfect the following cancer cells according to the steps in Example 1: human cervical cancer cells, human breast cancer cells (MCF7, BRCA009 or BRCA011), human endometrial cancer cells, lung adenocarcinoma cells, human pancreatic cancer cells, hepatocellular carcinoma cells., scarcoma cells, renal cell carcinoma cells, lymphoma cells, nasopharyngeal carcinoma cells, brain tumor cells, colon cancer cells, and leukemic cells. The protein transfection efficiency was assessed by flow cytometry or confocal microscope.

Fig. 8A-Fig. 8F are flow cytometry images showing the transfection efficiency of rhSTIP1 using HEPES buffer is about 98.8% in cervical cancer cells (Fig. 8A), 98.8% in breast cancer cells (Fig. 8B), 97% in endometrial cancer cells (Fig. 8C), 99.5% in lung adenocarcinoma cells (Fig. 8D), 99.9% in pancreatic cancer cells (Fig. 8E), and 96.6% in hepatocellular carcinoma cells (Fig. 8F).

Fig. 9A to Fig. 9F are immunofluorescent staining showing there is an increased Alexa Fluor 488 anti-mouse IgG signal using HEPES buffer (the right panel) compared to Opti-MEM (the left panel) in scarcoma cells (Fig. 9A), renal cell carcinoma (Fig. 9B), non Hodgkin's lymphoma (Fig. 9C), nasopharyngeal carcinoma (Fig. 9D), brain tumor (Fig. 9E), colon cancer (Fig. 9F), leukemia cells (Fig. 9G) and breast cancer cells (BRCA009 in Fig. 10A and BRCA011 in Fig. 10B).

### Example 7: Comparing HEPES buffer protein transfection efficiency with that of commercial protein transfection reagents

The protein transfection efficiency of HEPES was compared to other commercial reagents, such as PULsin™, ProteoJuice™, Pro-Ject™, Xfect™, and BioPORTER®. Briefly, anti-GAPDH antibody was transfected into ovarian cancer cells using HEPES buffer (20 mM) according to the steps in Example 1 or using various commercial transfection reagents. The transfected ovarian cancer cells were stained with Alexa Fluor 488 anti-mouse IgG antibodies and protein transfection efficiency was assessed by confocal fluorescent microscopy.

Fig. 11B to Fig. 11G show the IgG transfection efficiency of HEPES buffer (Fig. 11G) is comparable to that of the commercial protein transfection reagents such as PULSin™ (Fig. 11B), ProteoJuice™ (Fig. 11C), Pro-Ject™ (Fig. 11D), Xfect™ (Fig. 11E), and BioPorter® (Fig. 11F).

### Example 8: Evaluation of HEPES-mediated protein transfection pathway

An *in vitro* evaluation of HEPES-mediated protein transfection pathway was performed using the following endocytic inhibitors: sucrose (a clathrin-mediated endocytic inhibitor), filipin (a caveolae-mediated endocytic inhibitor) and amiloride (a macropinocytic inhibitor).

Human ovarian cancer cells were pretreated with Opti-MEM (the control group), 0.2-0.4 M sucrose, 5 µg/ml filipin or 0.0125-0.25 mM amiloride for 30 min, followed by the transfection of Alexa Fluor 488 anti-mouse IgG antibodies using 20 mM HEPES buffer according to the steps in Example 1.

The IgG antibody transfection efficiency was assessed by confocal fluorescent microscopy. Fig. 12A and 12B show all three inhibitors blocked the HEPES-mediated IgG transfection in human ovarian cancer cells.

The fluorescent signals of IgG were quantified by Q-Win software, expressed as a relative ratio in Fig. 12C and 12D (error bars indicate s.e.m. (n=3) and asterisk denotes significant difference, P< 0.001). All three inhibitors significantly reduced HEPES mediated IgG transfection. The HEPES mediated protein transfection is induced by at least one of the following pathways: clathrin-mediated or caveolae-mediated endocytosis, or macropinocytosis.

### Example 9: HEPES mediated anti-STIP1 transfection inhibits cell survival and induces cell death.

Fig. 13A shows the endogenous STIP1 expression in various ovarian cancer cell lines (TOV21G, MDAH2774, SKOV3 and ES2). The effects of anti-STIPl antibody and control mouse IgG2 antibodies in high STIP1 cancer cells (MDAH2774 and ES2) and low STIP1 cancer cells (TOV21G) were evaluated using HEPES buffer as the protein transfection composition according to the steps in Example 1. Cell viability at 24 hours and cytotoxicity at 48 h were analyzed with MTT and LDH assays, respectively.

STIP1 antibody transfection using HEPES buffer significantly inhibit cell growth and induced apoptosis in high STIP1 expression ovarian cancer cells (Fig. 13B for MADAH 2774 and Fig. 13C for ES2). STIP1 antibody transfection using HEPES buffer in lower STIP expression ovarian cancer cells did not affect cell viability or apoptosis (Fig. 13C for TOV21G). Fig. 13E and Fig. 13F show STIP1 antibody transfection in ovarian cancer cells using HEPES buffer according to the steps in Example 1 induced a higher expression of caspase 3 (an apoptotic marker) in high STIP1 expression ovarian cancer cells (Fig. 13E for ES2 and Fig. 13F for MDAH2774) compared to that of low STIP1 expression ovarian cancer cells (Fig. 13E for TOV21G).

Control mouse IgG2 antibodies or STIP1 antibodies were transfected into the following high STIP1 expressing cancer cells, using 20-30 mM of HPEPF buffer according to the steps in Example 1: endometrial cancer (RL952 cells), pancreatic cancer (BxPC3 cells), head and neck cancer (NPC-BM1 cells), lung cancer cells (CL1-0 cells), breast cancer (MCF7 cells), or colon cancer (HT29 cells). After 24 h or 48 h of incubation, cell viability (24 h) and cytotoxicity (48 h) were analyzed by MTT and LDH assays, respectively. Error bars indicate s.e.m. (n = 3). One, two, and three asterisks denote significant difference, P<0.05, <0.01, and <0.001, respectively.

Fig. 14A to Fig. 14F show STIP1 antibodies transfection significantly suppressed cell viability and increased cytotoxicity in endometrial cancer (Fig. 14A), pancreatic cancer (Fig. 14B), head and neck cancer (Fig. 14C), lung cancer (Fig. 14D), breast cancer5 (Fig. 14E), and colon cancer (Fig. 14F). These data suggest that the transfection of STIP1 antibody using HEPES buffer inhibits cancer cell proliferation and induces apoptosis.

## Claims

1. An *in vitro* method of delivering a protein to a cell, comprising:
(a) contacting the protein with a protein transfection composition to form a mixture, wherein the protein transfection composition consists of a 4-(2-hydroxyethyl)-1-piperazineethanesulfonic acid (HEPES) buffer; and
(b) contacting the cell with a minimal essential medium;
(c) contacting the cells of step (b) with the mixture of step (a).

2. The method of claim 1, wherein the protein is an antibody, preferably an anti-STIPl antibody.

3. The method of claim 1, wherein the protein is a recombinant protein or a peptide.

4. The method of claim 3, wherein the protein is rhSTIP1.

5. The method of claim 1, wherein the concentration of the HEPES buffer is between about 1 mM to about 50 mM.

6. The method of any of claims 1 to 5, wherein the HEPES buffer is substantially free of calcium phosphate.

7. The method of any of claims 1 to 6, wherein the cell is ovarian cancer cell, cervical cancer cell, breast cancer cell, endometrial cancer cell, lung cancer cell, pancreatic cancer cell, liver cancer cell, sarcoma cell, nasopharyngeal cancer cell, colon cancer cell, renal cancer cell, brain cancer cell, leukemia cell, or lymphoma cell.

8. The method of claim 7, wherein the cell is pancreatic cancer cell and the HEPES buffer is about 30 mM.

## Patentansprüche

1. Ein *in vitro* Verfahren zur Überbringung eines Proteins einer Zelle, umfassend
(a) Inkontaktbringen des Proteins mit einer Proteintransfektionszusammensetzung, um ein Gemisch zu bilden, wobei die Proteintransfektionszusammensetzung aus einem 4-(2-Hydroxyethyl)-1-piperazinethansulfonsäure-(HEPES)-Puffer besteht; und
(b) Inkontaktbringen der Zelle mit einem essentiellen Minimalmedium;
(c) Inkontaktbringen der Zellen von Schritt (b) mit dem Gemisch von Schritt (a).

2. Das Verfahren nach Anspruch 1, wobei das Protein ein Antikörper, vorzugsweise ein anti-STIP1-Antikörper ist.

3. Das Verfahren nach Anspruch 1, wobei das Protein ein rekombinantes Protein oder ein Peptid ist.

4. Das Verfahren nach Anspruch 3, wobei das Protein rhSTIP1 ist.

5. Das Verfahren nach Anspruch 1, wobei die Konzentration des HEPES-Puffers zwischen etwa 1 mM bis etwa 50 mM beträgt.

6. Das Verfahren nach einem der Ansprüche 1 bis 5, wobei der HEPES-Puffer im Wesentlichen frei von Calciumphosphat ist.

7. Das Verfahren nach einem der Ansprüche 1 bis 6, wobei die Zelle eine Eierstockkrebszelle, Gebärmutterhalskrebszelle, Brustkrebszelle, Endometriumkrebszelle, Lungenkrebszelle, Pankreaskrebszelle, Leberkrebszelle, Sarkomzelle, Nasenrachenkrebszelle, Kolonkrebszelle, Nierenkrebszelle, Hirnkrebszelle, Leukämiezelle oder Lymphomzelle ist.

8. Das Verfahren nach Anspruch 7, wobei die Zelle eine Pankreaskrebszelle ist und der HEPES-Puffer etwa 30 mM ist.

## Revendications

1. Méthode *in vitro* de délivrance d'une protéine à une cellule, comprenant :
(a) la mise en contact de la protéine avec une composition de transfection de protéine pour former un mélange, dans laquelle la composition de transfection de protéine consiste en un tampon acide 4-(2-hydroxyéthyl)-1-pipérazine-éthanesulfonique (HEPES) ; et
(b) la mise en contact de la cellule avec un milieu essentiel minimal ;
(c) la mise en contact des cellules de l'étape (b) avec le mélange de l'étape (a).

2. Méthode selon la revendication 1, dans laquelle la protéine est un anticorps, de préférence un anticorps anti-STIP1.

3. Méthode selon la revendication 1, dans laquelle la protéine est une protéine recombinante ou un peptide.

4. Méthode selon la revendication 3, dans laquelle la protéine est rhSTIP1.

5. Méthode selon la revendication 1, dans laquelle la concentration du tampon HEPES est comprise entre environ 1 mM et environ 50 mM.

6. Méthode selon l'une quelconque des revendications 1 à 5, dans laquelle le tampon HEPES est pratiquement exempt de phosphate de calcium.

7. Méthode selon l'une quelconque des revendications 1 à 6, dans laquelle la cellule est une cellule de cancer ovarien, une cellule de cancer du col, une cellule de cancer du sein, une cellule de cancer de l'endomètre, une cellule de cancer du poumon, une cellule de cancer pancréatique, une cellule de cancer du foie, une cellule de sarcome, une cellule de cancer naso-pharyngé, une cellule de cancer du côlon, une cellule de cancer rénal, une cellule de cancer cérébral, une cellule de leucémie, ou une cellule de lymphome.

8. Méthode selon la revendication 7, dans laquelle la cellule est une cellule de cancer pancréatique et le tampon HEPES est d'environ 30 mM.
